# EUROPEAN PATENT APPLICATION

(11) **EP 2 886 146 A1**
(43) Date of publication of application: **24.06.2015**
(21) Application number: 13199017.8
(22) Date of filing: 20.12.2013
(51) Int. Cl.: A61M 5/32

(54) **Needle safety device and drug delivery device**

(71) Applicant: Sanofi-Aventis Deutschland GmbH, 65929 Frankfurt am Main (DE)
(72) Inventor: The designation of the inventor has not yet been filed

(57) **Abstract**

The invention refers to a needle safety device (4) for a drug delivery device (1) comprising a needle cap remover (8) adapted to capture a needle cap (6) by a positive and/or non-positive connection, wherein the needle cap remover (8) comprises a top end (8.3), a needle shield (7) adapted to cover a needle (5) of the drug delivery device (2) before and after injection, wherein the needle shield (7) comprises a needle shield end (7.4) adapted to engage an injection site, and at least one blocking pin (9) which inwardly extends from the top end (8.3) and/or the needle shield end (7.4) into the needle cap remover (8) and/or into the needle shield (7).

## Description

The invention relates to a needle safety device for a drug delivery device, and to such a drug delivery device.

### Background of the Invention

Many drug delivery devices of the prior art, such as auto-injectors, syringes, have been developed for self-administration of the drug.

To protect the needle of the drug delivery device from damage or to protect people from needle-prick injuries before using of the device, the needle of the drug delivery device is covered by a protective needle cap of a flexible material which can be encased by a rigid, in particular plastic needle shield, the so-called rigid needle shield (shortly named RNS).

In order to prepare the drug delivery device for delivering a dose the protective needle cap has to be removed from the needle. This may be done by gripping the protective needle cap and pulling it away from the needle. This will usually result in an exposed needle which is undesirable in terms of needle safety or for person with a needle phobia. In order to solve that problem the needle of the drug delivery device could be covered by a needle shield or shroud in a manner to hide the needle when the protective needle cap is removed.

### Summary of the Invention

It is an object of the present invention to provide a needle safety device with a needle shield and a needle cap remover, wherein an unintended movement, in particular a movement of the needle shield before removing the needle cap remover, is prevented. Further, it is an object of the present invention to provide a drug delivery device with an improved needle safety.

The object is achieved by a needle safety device according to claim 1 and by a drug delivery device according to claim 11.

Preferred embodiments of the invention are given in the dependent claims.

According to the invention, a needle safety device for a drug delivery device comprises a needle cap remover adapted to capture a needle cap or a rigid needle shield encasing the needle cap by a positive and/or non-positive connection, wherein the needle cap remover comprises a top end, and a needle shield adapted to cover a needle of the drug delivery device before and after injection, wherein the needle shield comprises a needle shield end adapted to engage an injection or delivery site. The needle safety device comprises further at least one blocking pin. The blocking pin is a separate part which is adapted to be arranged at the needle shield of the drug delivery device. The at least one blocking pin is adapted to obstruct a guiding arrangement of the needle shield to prevent an unintended, in particular an early movement of the needle shield and thus an uncovering of the needle before using, in particular before injection but after removing the needle cap or of the encasing rigid needle shield by the needle cap remover. Thus, the invention ensures a protection from needle-prick injuries before and after using of the device, in particular also after removing the needle cap or the rigid needle shield encasing the needle cap and before injection.

Depending on the design of the needle safety device, the blocking pin can be arranged at the needle shield as a separate part wherein the blocking pin is carried through an opening in the needle shield end.

In a possible embodiment, the needle safety device comprises at least two blocking pins which are disposed opposite each other and are adapted to obstruct oppositely disposed guide arrangements of the needle shield of the drug delivery device. According to another feature of the invention, the needle cap remover and the blocking pin are provided as an integrated part. Preferably, the needle cap remover and the blocking pin/s are formed as an integrated injection moulded part.

According to another aspect of the invention, the blocking pin is carried through the top end of the needle safety device, in particular of the needle shield end, wherein an outside end of the blocking pin is adapted to form a button to unlock the guide arrangement during activation and/or injection process and thus to allow a movement of the needle shield at the earliest when the drug delivery device is placed onto the delivery site.

In a possible embodiment, the button is formed as a sticking out button. Alternatively, the button is formed as flat button, e.g. with spread wings. The sticking out button has the advantage that the risk of entrapment is lower than with the flat button by which an entrapment under the spread wings may be possible.

According to a further embodiment, the blocking pin comprises a recess adapted to a shape of a guide arm of the guiding arrangement of the needle shield. During activation movement of the drug delivery device and accordingly after pushing the button of the blocking pin, the guide arm slides into the recess of the blocking pin, thereby unlocking the guiding arrangement and moving the flexible guide arm together with the needle shield and thus together with the blocking pin.

For removing the protective needle cap, a gripping element, e.g. a gripping arm or a gripping label, is arranged at the top end of the needle cap remover. The gripping element is an integrated part of the needle cap remover and, in an assembled state of the drug delivery device, it extends through the opening of the needle shield outside the needle safety device and thus outside the drug delivery device.

According to a further embodiment, the blocking pin comprises a locking hook adapted to prevent a movement of the blocking pin and thus of the needle shield in a distal direction if the needle shield is in the extended position to avoid an uncovered needle after injection.

The invention also refers to a drug delivery device comprising a needle safety device as it is above described. The described needle safety device serves a safe and easy protection against an unintended, in particular a too early movement of the needle shield wherein the blocking pin is adapted to obstruct a guiding arrangement of the needle shield as long as the needle cap is not removed from the drug delivery device and the drug delivery device is not placed on an injection or delivery site.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows a perspective view of a drug delivery device with a needle safety device to cover a needle before and after injection,
- Figure 2: shows a partial side view of an embodiment of a needle safety device,
- Figure 3: shows a partial side view of another embodiment of a needle safety device,
- Figure 4: shows a partial side view of further embodiment of a needle safety device,
- Figure 5: shows a perspective view of another embodiment with a separate blocking component,
- Figure 6: shows a perspective view of a drug delivery device with a needle safety device with only one blocking pin, and
- Figure 7: shows an exploded view of a drug delivery device with a needle safety device with two blocking pins.

Corresponding parts are marked with the same reference symbols in all figures.

### Detailed Description of Preferred Embodiments

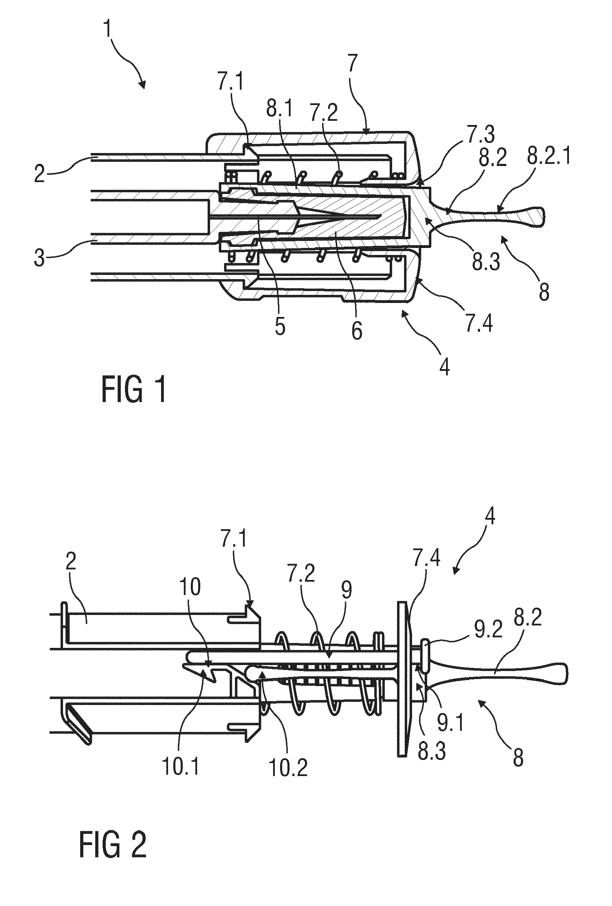

Figure 1 shows a perspective view of a drug delivery device 1 with a housing 2 containing and holding a container 3 and a needle safety device 4. The drug delivery device 1 could be an auto-injector with a container, an injection pen or a syringe, e.g. a pre-filled syringe, or another medicament container or any other application with comparable functional principle.

The container 3 such as, for example a syringe, is received in the housing 2. The housing 2 may be designed as a one part or multiple part housing, e.g. the housing 2 may be comprised a back housing part and a front housing part.

In the context of this specification, the term "back" or "proximal" end of a component or a device refers to the end closest to the user's hand or furthest away from the delivery or injection site and the term "front" or "distal" or "top" end of a component or a device refers to the end furthest from the user's hand or closest to the delivery or injection site.

In the embodiment, the housing 2 contains as a container 3 a syringe at which distal end a needle 5 may be fixed. Alternatively, the needle 5 may be removable therefrom, as a matter of design choice.

The needle safety device 4 comprises a needle cap 6, a needle shield 7 and a needle cap remover 8 which are adapted to be connected to each other by friction connection and/or positive locking connection and/or form-fitting connection.

As it can be seen, the needle shield 7 is being mounted on the housing 2 of the drug delivery device 1 in a movable manner by a snap-fit connection 7.1. A spring 7.2 biases at one end the needle shield 7 and at the other end the housing 2. The needle shield 7 is adapted to cover the needle 5 before and after injection, wherein the needle shield 7 comprises a needle shield end 7.4 adapted to engage an injection site.

As a matter of design choice, the needle shield 7 may be designed as a one part or in multiple parts wherein the needle shield end 7.4 may be designed as a one part separated from a lateral surface 7.5 encasing the needle 5.

The needle cap 6 is being mounted on the needle 5 in a separable manner. The needle cap 6 is designed as a protective rubber needle cap which covers the needle 5 before use to protect it against damages during transport or travel and to keep it sterile until injection starts. Alternatively, the needle cap 6 may be encased by a so called rigid needle shield (shortly named RNS). The needle cap 6 is of a typical form and is formed as an inner rubber needle shield or a soft rubber or rubber like core for easier and safer handling. The inner rubber needle shield is adapted to house and protect the needle 5.

The function and the arrangement of the needle cap remover 8 directly onto the needle cap 6 or onto the rigid needle shield encasing the needle cap 6 are identical and do not differ from each other. Thus, the following description regarding a needle cap remover arranged onto a rigid needle shield encasing a needle cap applies also to a needle cap remover directly arranged onto the needle cap without an encasing.

The needle cap remover 8 is of a typical form and comprises an inner portion 8.1, e.g. a tubular shaped portion, surrounding the needle cap 6 and an outer portion 8.2 extending outside through the needle shield 7. The needle cap remover 8 is adapted to capture the needle cap 6 thereby the inner portion 8.1 comprises at least one grip surface capable of gripping the needle cap 6 by a positive and/or non-positive locking connection in order to remove the needle cap 6. The outer portion 8.2 comprises at a distal or top end 8.3 a gripping portion 8.2.1 which is adapted to be arranged at the needle shield 7 and/or is formed as a gripping pin extending through an opening 7.3 of a distal needle shield end 7.4 and thus outside the needle shield 7 and might be attached to the needle cap 6.

After assembling of the drug delivery device 1 and in use, the needle cap remover 8 is pulled out of the opening 7.3 of the drug delivery device 1. Due to the coupling of the needle cap remover 8 and the needle cap 6, the needle cap 6 is removed from the needle 5 when the needle cap remover 8 is removed from the drug delivery device 1.

After removing of the needle cap 6, the drug delivery device 1 is pushed with its needle shield end 7.4 onto the injection or delivery site to provide an injection. To prevent an unintended, in particular a too early movement of the needle shield 7 when the needle cap 6 will be removed, at least one blocking pin 9 is provided at the needle safety device 4 which is shown in figures 2 to 6 in different embodiments.

For a better clarity, the following embodiments of the needle safety device 4 in figures 2 to 6 are shown without the lateral surfaces 7.5 of the needle shield 7, the container 3, the needle 5 and the needle cap 6.

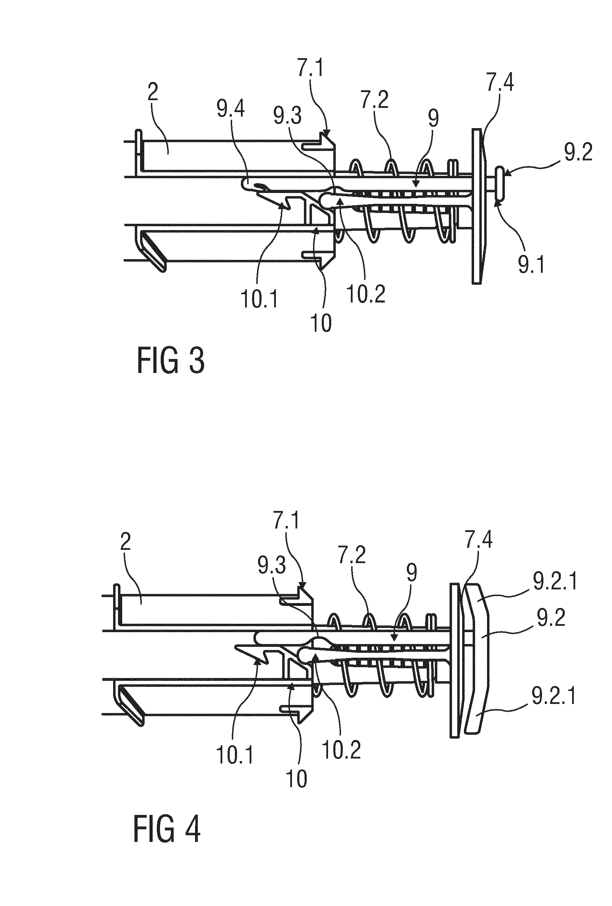

The needle safety device 4 comprises at least one blocking pin 9. The blocking pin 9 inwardly extends from the top end 8.3 of the needle cap remover 8 into a gap between the housing 2 and the needle shield 7 (= embodiment according to figure 2) and/or from the needle shield end 7.4 of the needle shield 7 into the needle shield 7 (=embodiments according to figures 3 to 6). The blocking pin 9 according to figure 2 has a pen-shape.

As it is shown in figure 2, the drug delivery device 1 comprises a guiding arrangement 10 for guiding the needle shield 7 between an extended position in which the needle 5 is covered or a retracted position in which the needle 5 extends through the opening 7.3 of the needle shield 7.

The guiding arrangement 10 comprises a guide track 10.1 and a corresponding guide arm 10.2 wherein one of the parts is arranged at the housing 2 and the other part is arranged at the needle shield 7.

The at least one blocking pin 9 is adapted to obstruct the guiding arrangement 10 to prevent an unintended, in particular a too early movement of the needle shield 7. According to the embodiment of figure 2, the blocking pin 9 is made of a rigid material and is arranged at the top end 8.3 of the needle cap remover 8. The blocking pin 9 extends from the top end 8.3 inwardly into the needle cap remover 8 and the needle shield 7 in such a manner that the rigid blocking pin 9 is arranged to the guiding arrangement 10, in particular to the guide track 10.1 as well as to the guide arm 10.2, in parallel when the needle shield 7 is in the extended position to obstruct the guide track 10.1 so that the flexible guide arm 10.2 cannot move into the guide track 10.1 and thus the guiding arrangement 10 is blocked.

If a user holds the drug delivery device 1 at the needle shield 7 during removal of the needle cap 6 by removing of the needle cap remover 8, the needle shield 7 will not trigger due to the arrangement of the blocking pin 9. Thus, an unintended, in particular a too early movement of the needle shield 7 is prevented.

If the needle cap remover 8 and thus with him the blocking pin 9 are removed, the guide track 10.1 will be free for the flexible guide arm 10.2 and thus the needle shield 7 may be movable from the extended position into the retracted position to provide an injection.

Depending on the design of the needle safety device 4, the blocking pin 9 can be arranged at the needle cap remover 8 and/or at the needle shield 7 wherein if the blocking pin 9 is arranged at the needle cap remover 8 then it is carried through the opening 7.3 in the needle shield end 7.4.

In a possible embodiment, the needle safety device 4 comprises at least two blocking pins 9 which are disposed opposite each other and are adapted to obstruct oppositely disposed guide arrangements 10 of the drug delivery device 1.

According to a further aspect of the invention, the needle cap remover 8 and the blocking pin 9 are provided as an integrated part. Preferably, the needle cap remover 8 and the blocking pin/s 9 are formed as an integrated injection moulded part, as it is shown in figure 2. Alternatively, the needle cap remover 8 and the blocking pin 9 are separate parts.

Figure 3 shows a partial side view of another embodiment of a needle safety device 4 with a blocking pin 9 which is carried through the needle shield end 7.4. An outside end 9.1 of the blocking pin 9 is adapted to form a button 9.2 to unlock the guide arrangement 10 during injection process and thus to allow a movement of the needle shield 7. The button 9.2 is formed as a sticking out button. The button 9.2 has to be pushed to unlock the guiding arrangement 10, wherein unlocking of the needle shield 7 will happen in the process of the injection.

Hereto, the blocking pin 9 comprises a recess 9.3 which corresponds to the shape of the guide arm 10.2 and thus gives place for the flexible guide arm 10.2 to slide in the recess 9.3, thereby the flexible guide arm 10.2 and the needle shield 7 can move together. In other words: During activation movement of the drug delivery device 1 and accordingly after pushing the button of the blocking pin 9, the guide arm 10.2 slides into the recess 9.3 of the blocking pin 9, thereby unlocking the guiding arrangement 10 and moving the flexible guide arm 10.2 together with the needle shield 7.

Figure 3 shows a partial side view of another embodiment of a needle safety device 4, wherein additionally to the recess 9.3 the blocking pin 9 comprises a locking hook 9.4 to lock the blocking pin 9 in the distal direction and thus in the extended position of the needle shield 7 (not shown).

Figure 4 shows a partial side view of further embodiment of a needle safety device 4.

The button 9.2 is alternatively formed as flat button with spread wings 9.2.1. The sticking out button without spread wings has the advantage that the risk of entrapment is lower than with the flat button 9.2 with spread wings 9.2.1 by which an entrapment under the spread wings 9.2.1 may be possible.

Depending on the design of the drug delivery device 1, the needle safety device 4 may be comprise more than one blocking pin 9, preferably two blocking pins 9 which dispose opposite to each other and to the respective oppositely disposed guiding arrangement 10 of the drug delivery device 1 for preventing the unintended use of the needle shield 7.

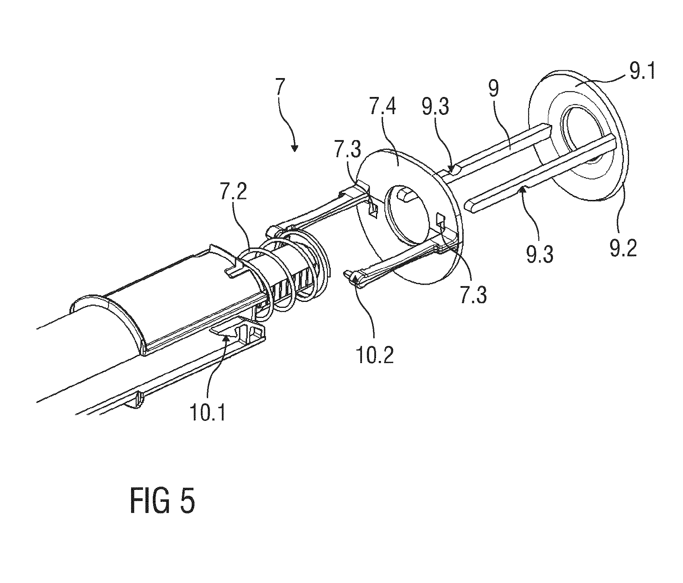

Figure 5 shows a perspective view of another embodiment with a separate arrangement of a blocking pin 9 onto the needle shield 7 of the drug delivery device 1.

According to this embodiment, the blocking pin 9 is part of a separate blocking component 11 which comprises a ring-shaped end 11.1 from which two blocking pins 9 extend. The needle shield end 7.4 comprises two corresponding holes 7.6 through which the blocking pins 9 are arranged and where appropriate are locked in an assembling state with the needle shield 7 so that during removing of the needle cap 6 by the needle cap remover 8 the blocking component 11 remains onto the needle shield 7. Otherwise, the blocking component 11 may be removed together with the needle cap remover 8 according to the embodiment of figure 2.

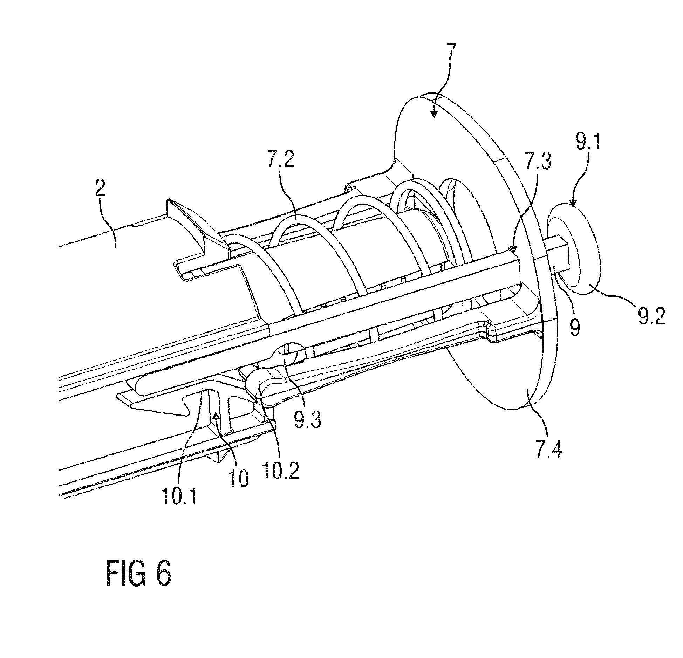

Figure 6 shows a perspective view of another embodiment with a separate arrangement of one blocking pin 9 onto the needle shield 7 of the drug delivery device 1. According to this embodiment, the blocking pin 9 remains onto the needle shield 7 after removing of the needle cap remover 8 and the needle cap 6. Further, one end of the blocking pin 9 is outwardly extended from the needle shield end 7.4 in a given distance and thus forms a button 9.2 so that if the drug delivery device 1 is placed onto a delivery site, the blocking pin 9 is pushed onto the needle shield end 7.4 and inside the needle shield 7 as it is described above for the embodiment according to figure 3.

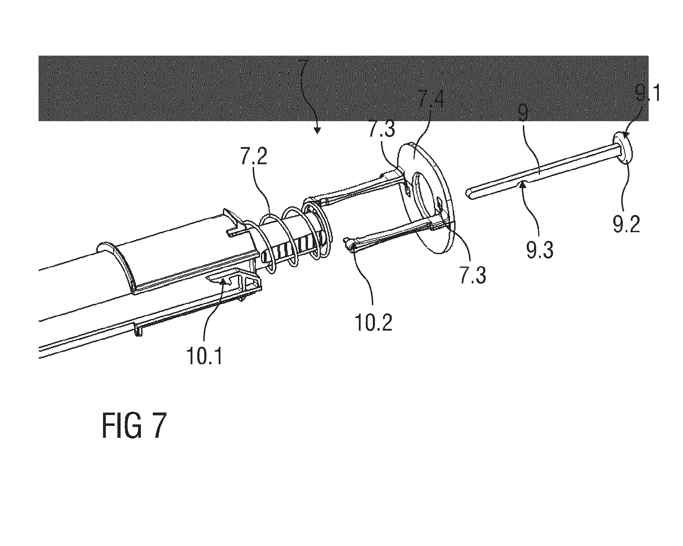

Figure 7 shows an exploded view of a drug delivery device 1 similar to the drug delivery device 1 according to figure 6. The drug delivery device 1 of figure 7 comprises two separate blocking pins 9. The arrangement and functions are similar to the arrangement and function of the only one blocking pin 9 in figure 6.

With the integrated blocking pin 9 which is integrated part of the needle cap remover 8 (= embodiment of figure 2) and/or which is separately arranged onto the needle shield 7 (= embodiments of figures 3 to 6) an easy, smart and safe needle safety device 4 is provided which may be easy assembled to a drug delivery device 1 and which easily prevents an unintended, in particular a too early movement of the needle shield 7.

Thus, the described needle safety device 4 serves a safe and easy protection against an early movement of the needle shield 7 wherein the blocking pin 9 is adapted to obstruct the guiding arrangement 10 of the needle shield 7.

The term "drug" or "medicament", as used herein, means a pharmaceutical formulation containing at least one pharmaceutically active compound,

wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,

wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,

wherein in a further embodiment the pharmaceutically active compound comprises at least one peptide for the treatment and/or prophylaxis of diabetes mellitus or complications associated with diabetes mellitus such as diabetic retinopathy,

wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: Drug delivery device
- 2: Housing
- 3: Container
- 4: Needle safety device
- 5: Needle
- 6: Needle cap
- 7: Needle shield
- 7.1: Snap-fit connection
- 7.2: Spring
- 7.3: Opening
- 7.4: Needle shield end
- 7.5: Lateral surface
- 8: Needle cap remover
- 8.1: Inner portion
- 8.2: Outer portion
- 8.2.1: Gripping portion
- 8.3: Top end
- 9: Blocking pin
- 9.1: Outside end
- 9.2: Button
- 9.2.1: Spread wings
- 9.3: Recess
- 9.4: Locking hooks
- 10: Guiding arrangement
- 10.1: Guide track
- 10.2: Guide arm
- 11: Blocking component
- 11.1: Ring-shaped end

## Claims

1. Needle safety device (4) for a drug delivery device (1), comprising:
- a needle cap remover (8) adapted to capture a needle cap (6) by a positive and/or non-positive connection, wherein the needle cap remover (8) comprises a top end (8.3),
- a needle shield (7) adapted to cover a needle (5) of the drug delivery device (2) before and after injection, wherein the needle shield (7) comprises a needle shield end (7.4) adapted to engage an injection site, and
- at least one blocking pin (9) which inwardly extends from the top end (8.3) into the needle cap remover (8) and/or into the needle shield (7).

2. Needle safety device (4) according to claim 1, wherein
at least two oppositely disposed blocking pins (9) are provided.

3. Needle safety device (4) according to claim 2, wherein
the needle cap remover (8) and the blocking pin (9) are provided separately or as an integrated part.

4. Needle safety device (4) according to any of the preceding claims, wherein
the blocking pin (9) is carried through the top end (8.3) and/or the needle shield end (7.4) wherein an outside end (9.1) of the blocking pin (9) is adapted to form a button (9.2).

5. Needle safety device (4) according to claim 4, wherein
the button (9.2) is formed as a sticking out button.

6. Needle safety device (4) according to claim 4, wherein
the button (9.2) is formed as flat button.

7. Needle safety device (4) according to claim 5 or 6, wherein
the blocking pin (9) comprises a recess (9.3) adapted to a shape of a guide arm (10.2) of the needle shield (7).

8. Needle safety device (4) according to any of the preceding claims, wherein
a gripping portion (8.2.1) is arranged at the top end (8.3) of the needle cap remover (8).

9. Needle safety device (4) according to any of the preceding claims, wherein
the blocking pin (9) comprises a locking hook (9.4) adapted to lock the blocking pin 9 in a distal direction.

10. Drug delivery device (1) comprising a needle safety device (4) according to any of the preceding claims.
